# EUROPEAN PATENT SPECIFICATION

(11) **EP 4 208 232 B1**
(45) Date of publication and mention of the grant of the patent: **13.11.2024**
(21) Application number: 21773000.1
(22) Date of filing: 31.08.2021
(51) Int. Cl.: A61M 15/08, A61M 11/00, A61M 15/00

(54) **RELOADABLE MULTI-DOSE DRUG DELIVERY DEVICE**
WIEDERLADBARE MEHRFACHDOSIS-ARZNEIMITTELABGABEVORRICHTUNG
DISPOSITIF D'ADMINISTRATION DE MÉDICAMENT MULTIDOSE RECHARGEABLE

(30) Priority: 01.09.2020 US 202063072983 P
(43) Date of publication of application: 12.07.2023
(73) Proprietor: JANSSEN Pharmaceutica NV, 2340 Beerse (BE)
(72) Inventor: CANNAMELA, Michael, New York, New York 10001 (US); CASSEBEE, Jimmy Vinh Hoang, Milpitas, California 95035 (US); YAN, Hong, Milpitas, California 95035 (US); SPERRY, Christina, Boston, Massachusetts 02111 (US); PEREZ, Dolores, Easton, Pennsylvania 18045 (US); LARSON, Chaley John, Horsham, Pennsylvania 19044 (US); VESOLE, Steven M., Milpitas, California 95035 (US); HUBERT, Emma Louise, Milpitas, California 95035 (US)
(74) Representative: Carpmaels & Ransford LLP
(86) International application number: PCT/EP2021/074032
(87) International publication number: WO 2022/049076

(56) References cited:
- WO-A2-02/13886
- US-A1- 2011 233 232
- US-A1- 2016 325 080
- US-A1- 2019 015 613
- US-A1- 2020 197 630
- US-B1- 6 708 846

## Description

### FIELD

The present disclosure relates generally to reloadable drug vials for multi-dose delivery and drug products utilizing the same.

### BACKGROUND

There are many different ways in which a drug can be administered to a user. Depending on the drug, intranasal drug delivery can be one of the most effective ways to achieve desired clinical benefits in a timely manner and in a manner that is convenient and comfortable for a patient.

Intranasal drug administration is a non-invasive route for drug delivery. Since the nasal mucosa offers numerous benefits as a target tissue for drug delivery, a wide variety of drugs may be administered by intranasal systemic action. Moreover, intranasal drug delivery can avoid the risks and discomfort associated with other routes of drug delivery, such as intravenous drug delivery, and can allow for easy self-administration.

Generally, to maximize the efficacy of the drug through intranasal administration, the majority volume of the aerosolized dose of the drug needs to reach the correct region of the nasal cavity. As such, additional measures may need to be taken for effective intranasal drug delivery. For example, the user may need to have a clear nostril, tilt their head back at approximately 45°, close the opposite nostril, and then sniff gently while the dose of drug is administered. In order to coordinate these measures, and given that nasal administration is intimate, self-administration by the user may be desired. Further, due to the nasal cycle (alternating physiological partial congestion of the nasal turbinate to facilitate nasal function) or pathological congestion, one nostril is likely to provide a more effective drug delivery route than the other nostril at any given time. As such, it is desired that an equal dose of the drug be delivered to each nostril of the user to inhibit under-dosing of the drug.

Dual-dose intranasal drug delivery devices are available that are designed for self-administration of two distinct aerosolized sprays, one for each nostril, that together constitute one dose of drug. Such drug delivery devices require a series of operational steps that the user needs to properly carry out to effect optimal drug delivery through self-administration. After the drug is delivered, the device may be disposed of as a used device having no drug remaining in the device or having a residual amount of the drug remaining in the device that cannot be further delivered from the device. Disposing the device after use may have adverse environmental impacts by generating waste that cannot be recycled, that is accidentally not recycled, or that cannot be efficiently recycled from the particular geographic region of the device's use. Some nasal drug delivery devices are designed for one-time use to deliver one dose of drug, such as for prescription drugs where the amount of drug delivered to the patient is important and is typically prescribed at a certain amount per dose. Such one-time use devices may therefore have exacerbated adverse environmental impacts since they are not reused before disposal.

Additionally, one-time use nasal drug delivery devices prevent patients from becoming familiar and comfortable with using the same device for multiple drug deliveries. Even if each sequentially used nasal drug delivery device is the same as one another, at least some patients may not recognize each device's similarity and/or may feel reluctance and/or nervousness at using a device they have never used before.

Accordingly, there remains a need for improved nasal drug delivery devices.

In WO0213886A2, there is described an apparatus and method for the self-administration of a plurality of doses of an intranasal liquid pharmaceutical composition, including opioid analgesics, that includes a drug delivery device containing a plurality of sealed vials, each vial containing a predetermined volume of the pharmaceutical composition, a pump assembly for conveying the liquid pharmaceutical composition from the interior of the vial and discharging it as a nasal spray in response to manual activation by the patient, and programmable means for sequentially advancing a vial to the ready position after passage of a prescribed time interval following the last activation of the delivery device.

In US2016325080A1, there is described a method of treatment and a nasal delivery device for dosages containing dissolved gaseous carbon dioxide in saline fluid for treating head ailments.

In US2020197630A1, there is described a dispenser device for fluid or powder, including an air expeller and a reservoir.

In US6708846B1, there is described a dispenser, especially a disposable atomizer, with a dispenser unit.

### SUMMARY

In general, reloadable drug vials for multi-dose delivery, drug products utilizing the same, and methods of using reloadable drug vials for multi-dose delivery are provided.
The invention is defined by claim 1. Further embodiments are defined by the dependent claims.

### BRIEF DESCRIPTION OF DRAWINGS

The present invention is described by way of reference to the accompanying figures which are as follows:
FIG. 1 is a side cross-sectional view of one embodiment of a vial;
FIG. 2 is a block diagram of one embodiment of a drug delivery device configured to releasably couple to a vial;
FIG. 3 is a block diagram of one embodiment of the drug delivery device of FIG. 2;
FIG. 4 is a side view of one embodiment of the drug delivery device of FIG. 3;
FIG. 5 is a partial, side cross-sectional view of the drug delivery device of FIG. 4;
FIG. 6 is a side view of the drug delivery device of FIG. 4 coupled to one embodiment of the vial of FIG. 2;
FIG. 6A is a side view of the vial of FIG. 6;
FIG. 7 is a side view of another embodiment of the drug delivery device of FIG. 3;
FIG. 8 is a side view of the drug delivery device of FIG. 8 coupled to the vial of FIG. 6;
FIG. 9 is a side view of still another embodiment of the drug delivery device of FIG. 3;
FIG. 10 is a side view of yet another embodiment of the drug delivery device of FIG. 3;
FIG. 11 is a side view of still another embodiment of the drug delivery device of FIG. 3 and of one embodiment of a cartridge strip coupled to a plurality of vials;
FIG. 12 is a side view of another embodiment of a cartridge strip coupled to a plurality of vials;
FIG. 13 is a side view of yet another embodiment of a cartridge strip coupled to a plurality of vials and to another embodiment of the drug delivery device of FIG. 3;
FIG. 14 is a front view of the cartridge strip, vials, and drug delivery device of FIG. 13;
FIG. 15 is a top view of one of vials and a portion of the cartridge strip of FIG. 13;
FIG. 16 is a front view of the cartridge strip, vials, and drug delivery device of FIG. 13 with a user's fingers and thumb positioned relative thereto;
FIG. 17 is a front view of the cartridge strip, vials, and drug delivery device of FIG. 16 with the vial moved proximally relative to the cartridge strip and the vial;
FIG. 18 is a side view of the cartridge strip, vials, and drug delivery device of FIG. 17;
FIG. 19 is a side view of the cartridge strip, vials, and drug delivery device of FIG. 17 with the vial moved distally relative to the cartridge strip and the vial;
FIG. 20 is a side view of the cartridge strip, vials, and drug delivery device of FIG. 19 with the drug delivery device positioned at a different position relative to the cartridge strip and the vials; and
FIG. 21 is a side cross-sectional view of another embodiment of a vial.

### DETAILED DESCRIPTION

Certain exemplary embodiments will now be described to provide an overall understanding of the principles of the structure, function, manufacture, and use of the devices, systems, and methods disclosed herein. One or more examples of these embodiments are illustrated in the accompanying drawings. A person skilled in the art will understand that the devices, systems, and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

Further, in the present disclosure, like-named components of the embodiments generally have similar features, and thus within a particular embodiment each feature of each like-named component is not necessarily fully elaborated upon. Additionally, to the extent that linear or circular dimensions are used in the description of the disclosed systems, devices, and methods, such dimensions are not intended to limit the types of shapes that can be used in conjunction with such systems, devices, and methods. A person skilled in the art will recognize that an equivalent to such linear and circular dimensions can easily be determined for any geometric shape. A person skilled in the art will appreciate that a dimension may not be a precise value but nevertheless be considered to be at about that value due to any number of factors such as manufacturing tolerances and sensitivity of measurement equipment. Sizes and shapes of the systems and devices, and the components thereof, can depend at least on the size and shape of components with which the systems and devices will be used.

Various exemplary reloadable drug vials for multi-dose delivery, drug products utilizing the same, and methods of using reloadable drug vials for multi-dose delivery are provided. In general, a nasal drug delivery device is configured to releasably couple to a first drug holder that contains a drug therein to be dispensed by the drug delivery device. After the drug is delivered from the drug delivery device, the first drug holder is configured to be removed from the drug delivery device and replaced with a second drug holder containing a drug therein to be dispensed by the drug delivery device. The same drug delivery device is therefore configured to deliver multiple doses. After the drug is delivered from the drug delivery device, the drug delivery device and the second drug holder releasably coupled thereto can be disposed of as waste. Alternatively, after the drug is delivered from the drug delivery device, the second drug holder can be removed from the drug delivery device and replaced with a third drug holder containing a drug therein to be dispensed by the drug delivery device and so on with one or more additional drug holders sequentially being coupled to and used in delivering a drug from the drug delivery device.

Replacing a drug holder after drug delivery allows a remainder of the drug delivery device to be re-used, thereby generating less waste than drug delivery devices that are disposed of after use with the drug holder used with the drug delivery device. Replacing the drug holder after drug delivery allows for one drug delivery device to be purchased that can be used for a plurality of dose deliveries instead of requiring multiple drug delivery devices with pre-loaded drug holders to be purchased to deliver that same number of doses, thereby reducing health care expenses since fewer drug delivery devices need to be purchased. Replacing the drug holder after drug delivery allows for the same drug delivery device to be repeatedly used by the same user for a plurality of dose deliveries, which may allow for the user to develop familiarity and confidence in the device over time and/or may reduce user reluctance and/or nervousness in using the device since (after the first use) they are not using a device they have never used before.

In an exemplary embodiment, the drugs in each of the drug holders configured to be releasably coupled to the drug delivery device are the same such that the drug delivery device is usable to deliver subsequent doses of the same drug to a user of the drug delivery device. The user may therefore be able to use the same drug delivery device repeatedly in fulfilling a drug prescription. In other embodiments, the drugs in each of the drug holders configured to be releasably coupled to the drug delivery device are not all the same such that the drug delivery device is usable to deliver doses of different drugs to a user of the drug delivery device. The user may therefore be able to use the same drug delivery device repeatedly in fulfilling two or more drug prescriptions. In embodiments in which the drugs in each of the drug holders are not all the same, at least one of the drug holders contains a first drug therein and at least one of the drug holders contains a second drug that is different than the first drug. In embodiments in which at least three drug holders are provided for releasable coupling to the drug delivery device, the number of different drugs contained in the various drug holders can be more than two.

The drug to be delivered using a drug delivery device as described herein can be any of a variety of drugs. Examples of drugs that can be delivered using a drug delivery device as described herein include antibodies (such as monoclonal antibodies), hormones, antitoxins, substances for the control of pain, substances for the control of thrombosis, substances for the control of infection, peptides, proteins, human insulin or a human insulin analogue or derivative, polysaccharide, DNA, RNA, enzymes, oligonucleotides, antiallergics, antihistamines, anti-inflammatories, corticosteroids, disease modifying anti-rheumatic drugs, erythropoietin, and vaccines. Examples of drugs that can be delivered using a drug delivery device as described herein include ketamine (e.g., Ketalar^{®}), esketamine (e.g., Spravato^{®}, Ketanest°, and Ketanest-S°), naloxone (e.g., Narcan°), and sumatriptan (e.g., Imitrex^{®}).

In an exemplary embodiment, the drug holder configured to be releasably coupled to the drug delivery device is a vial. An exemplary vial is formed of one or more materials, e.g., glass, polymer(s), etc. In some embodiments, a vial can be formed of glass. In other embodiments, a vial can be formed of one or more polymers. In yet other embodiments, different portions of a vial can be formed of different materials.

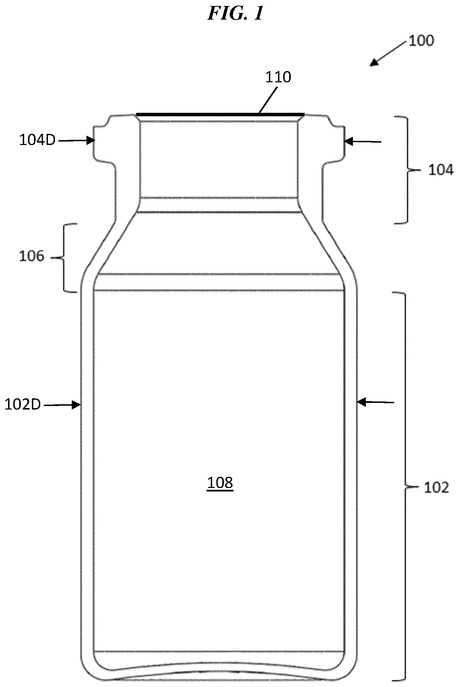

FIG. 1 illustrates one exemplary embodiment of a drug holder in the form of a vial 100 configured to contain a drug therein. The vial 100 includes a base or distal portion 102 and a head or proximal portion 104. As shown, the vial 100 also includes an inwardly tapering neck portion 106 that extends between the base portion 102 and the head portion 104. The inwardly tapering neck portion 106 allows the head portion 104 to have a maximum outer diameter 104D that is less than a maximum outer diameter 102D of the base portion 102. The head portion 104 having a smaller maximum outer diameter 104D than the base portion's maximum outer diameter 102D may ease coupling of the vial 100 to a drug delivery device in embodiments in which a user holds the base portion 102 and inserts the vial 100 proximally into the drug delivery device by providing a larger area of the vial 100 for the user to hold (e.g., the base portion 102) and a smaller area of the vial 100 to lead the insertion of the vial 100 into the drug delivery device (e.g., the head portion 102). In other embodiments, the taper between the base and head portions 102, 104 can be omitted, and the base and head portions 102, 104 can have a same maximum outer diameter 102D, 104D as one another.

The base portion 102 defines a hollow interior 108 within the base portion 102 that is configured to contain the drug therein. In an exemplary embodiment, an amount of the drug in the vial 100 is such that the drug can be contained entirely within the hollow interior 108 of the base portion 102, but the amount of the drug can be such that drug entirely fills the hollow interior 108 of the base portion 102 and also fills at least some part of the neck portion 106 and optionally also at least some part of the head portion 104. While the base portion 102 can have a variety of configurations, in this illustrated embodiment, the base portion 102 has a substantially cylindrical shape. In other embodiments, the base portion 102 can have any other suitable shapes, e.g., a substantially rectangular shape, etc. A person skilled in the art will appreciate that a shape may not be a precise shape (e.g., a precise cylinder or a precise rectangle) but nevertheless be considered to be substantially that shape due to any number of factors, such as manufacturing tolerances and sensitivity of measurement equipment.

The vial 100 includes a seal member 110 in the form of a septum at a proximal end thereof in the head portion 104. The seal member 110 is configured to provide a fluid tight seal such that the drug is contained in the vial 100 until the seal provided by the seal member 110 is broken. The seal provided by the seal member 110 can be broken in a variety of ways, such as by being pierced by a needle of the drug delivery device to which the vial 100 is releasably coupled. The seal member 110 can have a variety of configurations, as will be appreciated by a person skilled in the art, such as by being a pierceable polymer septum or a foil layer. The seal member 110 can be protected from accidental puncturing or piercing before intended use with a removable protective member or stopper, such as a TE seal, etc. In some embodiments, the seal member 110 can be omitted and instead a removable protective member or stopper can be provided that is removed just prior to use of the vial 100.

An exemplary vial can include a variety of features to facilitate sealing and storing a drug therein, as described herein and illustrated in the drawings. However, a person skilled in the art will appreciate that the vials can include only some of these features and/or can include a variety of other features known in the art. The vials described herein are merely intended to represent certain exemplary embodiments.

A drug delivery device configured to releasably couple to a vial can have a variety of configurations. In general, the drug delivery device is configured to deliver a drug to a patient, where the drug is provided in a defined dosage form within the drug delivery device.

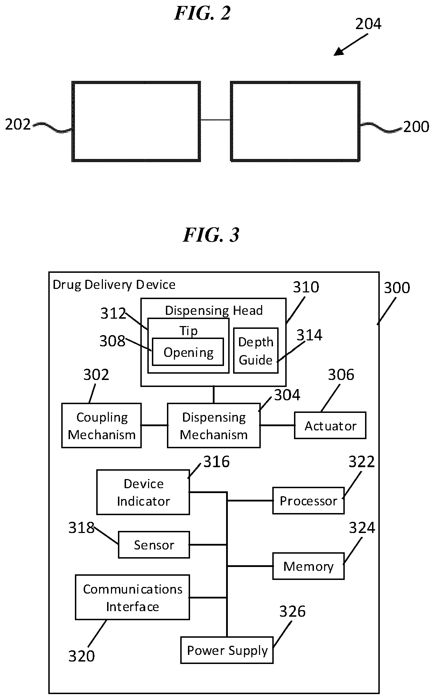

FIG. 2 illustrates one embodiment of a drug delivery system 204 including a drug delivery device 200 configured to releasably couple to a vial 202, such as the vial 100 of FIG. 1. As will be appreciated by a person skilled in the art, the drug delivery device 200 and the vial 202 can each include different features in different embodiments depending upon various requirements, such as the type of drug, typical dosage(s) of the drug, safety requirements in various jurisdictions, whether the device is powered, etc.

FIG. 3 illustrates an exemplary embodiment of the drug delivery device 200 of FIG. 2. FIG. 3 illustrates a drug delivery device 300 that includes a coupling mechanism 302 configured to releasably couple to a vial, such as the vial of FIG. 1. The coupling mechanism 302 can have a variety of configurations, as discussed further below.

The drug delivery device 300 also includes a dispensing mechanism 304 that is configured to be operatively coupled to the vial releasably coupled to the coupling mechanism 302 and that is configured to release a drug from the drug delivery device 300, e.g., from the vial releasably coupled to the coupling mechanism 302, so that the drug can be administered to a patient. The dispensing mechanism 304 can have a variety of configurations. For example, the dispensing mechanism 304 can include a piston and/or a needle configured to pierce through or puncture a seal member of the vial releasably coupled to the drug delivery device 300.

The drug delivery device 300 also includes an actuator 306 configured to be actuated by a user to cause the dispensing mechanism 304 to begin delivering a dose of the drug through an opening or nozzle 308 in the drug delivery device 300. In an exemplary embodiment, the drug delivery device 300 is configured to be self-administered such that the user who actuates the actuator 306 is the patient receiving the drug from the drug delivery device 300.

The opening 308 through which the drug exits the drug delivery device 300 is formed in a dispensing head 310 of the drug delivery device 300 in a tip 312 of the dispensing head 310. The tip 312 is configured to be inserted into a nostril of a patient. In an exemplary embodiment, the tip 312 is configured to be inserted into a first nostril of the patient during a first stage of operation of the drug delivery device 300 and into a second nostril of the patient during a second stage of operation of the drug delivery device 300. The first and second stages of operation involve two separate actuations of the actuator 306, a first actuation corresponding to a first dose of the drug being delivered and a second actuation corresponding to a second dose of the drug being delivered. The dispensing head 310 includes a depth guide 314 configured to contact skin of the patient, e.g., between the patient's first and second nostrils, such that a longitudinal axis of the dispensing head 310 is substantially aligned with a longitudinal axis of the nostril in which the tip 312 is inserted. A person skilled in the art will appreciate that the longitudinal axes may not be precisely aligned but nevertheless be considered to be substantially aligned due to any number of factors, such as manufacturing tolerances and sensitivity of measurement equipment.

In an exemplary embodiment, the dispensing head 310 has a tapered shape in which the dispensing head 310 has a smaller diameter at its distal end than at its proximal end where the opening 308 is located. The opening 308 having a relatively small diameter facilitates spray of the drug out of the opening 308, as will be appreciated by a person skilled in the art. A spray chamber through which the drug is configured to pass before exiting the opening 308 is located within a proximal portion of the tapered dispensing head 310, distal to the opening 308. When the drug passes through the spray chamber at speed, the spray chamber facilitates production of a fine mist that exits through the opening 308 with a consistent spray pattern.

The drug delivery device 300 also includes a device indicator 316 configured to present information to a user about a status of the drug delivery device 300 and/or the drug contained in the vial releasably coupled to the drug delivery device 300. The device indicator 316 can be a visual indicator, such as a display screen, one or more lights, one or more colored and/or numbered markings, etc. Alternatively or in addition, the device indicator 316 can be an audio indicator configured to provide sound.

The drug delivery device 300 also includes a sensor 318 configured to sense information relating to the drug delivery device 300 and/or the drug contained in the vial releasably coupled to the drug delivery device 300. Examples of information that the sensor 318 can sense include environmental conditions (e.g., temperature, humidity, geographic location, time, etc.) and drug holder status (e.g., whether or not a vial is releasably coupled to the coupling mechanism 302). The drug delivery device 300 can also include a communications interface 320 configured to communicate externally data which has been gathered by the sensor 318 about the drug delivery device 300 and/or the drug contained in the vial releasably coupled to the drug delivery device 300, which may facilitate analysis regarding the patient's treatment, patient compliance, use of the drug delivery device 300, etc.

In embodiments in which the drug delivery device 300 includes one or more electrical components, e.g., the device indicator 316 (which in some embodiments can be powered while in other embodiments not be powered), the sensor 318, the communications interface 320, a processor 322, a memory 324, etc., the drug delivery device 300 includes a power supply 326 configured to deliver electrical power to the one or more electrical components of the drug delivery device 300. The power supply 326 can be a source of power which is integral to drug delivery device 300 and/or can be a mechanism configured to connect the drug delivery device 300 to an external source of power. The processor 322 is configured to receive gathered data from the sensor 318 and to cause the data to be stored in the memory 324, to be indicated on the device indicator 310, and/or and to be communicated externally via the communications interface 320. The memory 324 is configured to store instructions that are configured to be executed by the processor 322 for the processor 322 to process information regarding the various electrical components with which the processor 322 is in communication.

As mentioned above, the drug delivery device 300 can include different features in different embodiments depending upon various requirements. For example, the drug delivery device 300 can omit any one or more of the depth guide 314, the device indicator 316, the sensor 318, the communications interface 320, the processor 322, the memory 324, and the power supply 326.

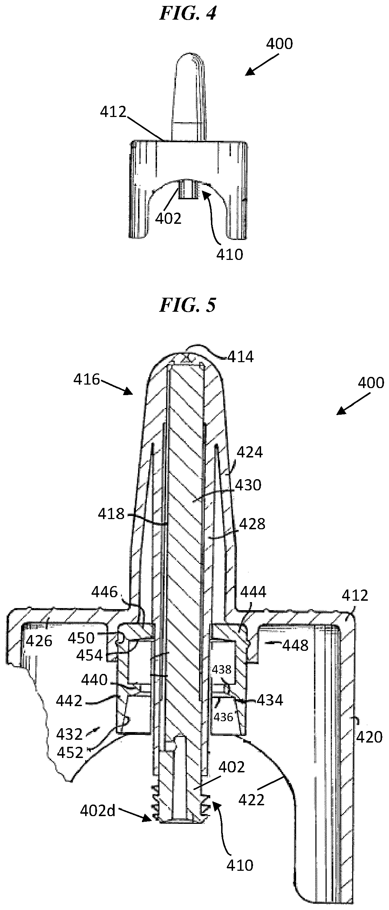

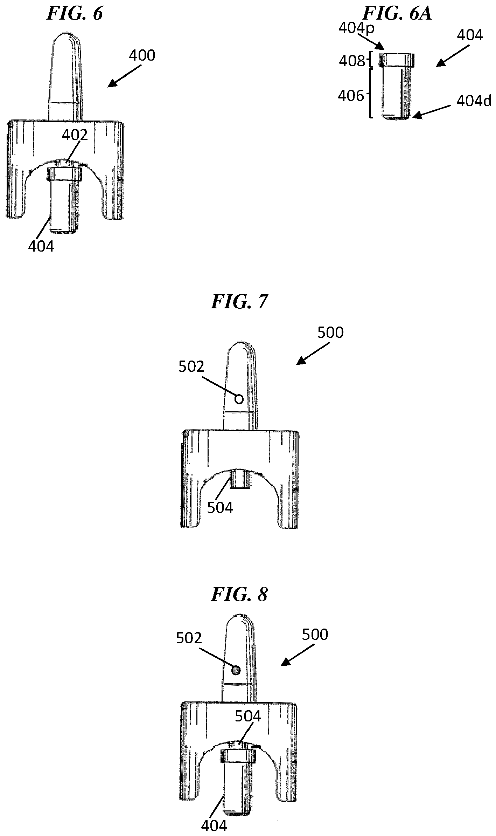

FIGS. 4-6 illustrate an exemplary embodiment of the drug delivery device 300 of FIG. 3. FIGS. 4-6 illustrate a drug delivery device 400 that includes a coupling mechanism 402 configured to releasably couple to a vial 404. FIG. 6 illustrates the vial 404 releasably coupled to the drug delivery device 400. FIG. 6A illustrates the vial 404 as a standalone element. The vial 404 is generally configured and used similar to the vial 100 of FIG. 1. The vial 404 in this illustrated embodiment includes a base portion 406 and a head portion 408 that has a larger maximum outer diameter than then base portion 406, though the vial 404 does not include a tapering neck portion. The vial 404 also includes a seal member at a proximal end thereof in the head portion 408, although as mentioned above, in some embodiments, the seal member can be omitted.

The coupling mechanism 402 in this illustrated embodiment includes threading 410 on an external surface of a piston of the drug delivery device 400 that is configured to releasably thread with threading on an internal surface of the vial 404. The vial's threading is obscured in FIGS. 6 and 6A. The piston defines the coupling mechanism 402. The coupling mechanism 402 can have other embodiments. For example, the coupling mechanism 402 can include a clip configured to releasably clip to the head portion 408 of the vial 404. For another example, one of the coupling mechanism 402 and the vial 404 can include one or more protrusions extending therefrom configured to be releasably seated in one or more corresponding grooves or depressions formed in the other of the coupling mechanism 402 and the vial 404. In some embodiments, the one of the coupling mechanism 402 and the vial 404 that includes the one or more protrusions can be configured to flex at least in an area in which the one or more protrusions are located to facilitate seating of the one or more protrusions in, and decoupling of the one or more protrusions from, the one or more corresponding grooves or depressions. In some embodiments, the one or more protrusions and the one or more corresponding grooves or depressions can be configured similar to a quarter-turn cam latch or lock.

A distal end 402d of the piston 402 is configured to puncture or pierce through the vial's seal member. In other words, as the vial 404 and the drug delivery device 400 are coupled, e.g., threaded, together, the piston 402 punctures or pierces through the vial's seal member. The vial 404 can thus be fluid tight until the vial 404 is coupled to the drug delivery device 400, after which the drug contained in the vial 404 can flow out of the vial 404 for delivery. The distal end 402d of the piston 402 can include a sharp edge, e.g., around a perimeter of the piston's distal end 402d, to facilitate the puncturing or piercing of the vial's seal member or can otherwise include a feature configured to facilitate the puncturing or piercing, such as by having one or more sharp extensions extending distally therefrom. In some embodiments, the seal member can be omitted from the vial 404, in which case the piston 402 need not be configured to puncture or pierce through the vial's seal member.

The drug delivery device 400 includes a body 412 in the form of a casing. The vial 404 is configured to move relative to the body 412 to cause delivery of the drug from the drug delivery device 400 through an opening 414 formed in a tip 416 of the body 412. The tip 416 is configured to be inserted into a first nostril of the patient during a first stage of operation of the drug delivery device 400 and into a second nostril of the patient during a second stage of operation of the drug delivery device 400. The vial 404 thus defines an actuator configured to be actuated to cause drug delivery to begin. The drug in this illustrated embodiment is configured to be self-administered such that the user who actuates the actuator, e.g., the vial 404, is the patient receiving the drug from the drug delivery device 400.

To allow the vial 404 to be actuated after being threaded to the piston 402 such that the vial 404 moves proximally relative to the piston 402, the vial 404 is threaded proximally past the threading 410 on the piston 402. The vial 404 can thereafter be pushed proximally to actuate the drug delivery device 400. It will be appreciated that the terms "proximal" and "distal" are used herein with reference to a user gripping a nasal drug delivery device for drug delivery, with proximal indicating a direction toward the nose. However, drug delivery devices are used in many orientations and positions, and these spatial terms are not intended to be limiting and absolute.

The drug delivery device 400 does not need to be primed after the vial 404 has been releasably coupled thereto and prior to actuation of the drug delivery device 400 to cause drug delivery, which may ease use of the drug delivery device 400 by a user.

The body 412 has the piston 402 fixed thereto. From an inner end face of the piston 402 emanates an outlet channel 418, which is guided in valve-free manner to the opening 414 leading into the open and which is formed by an atomizing nozzle. The outlet channel 418 becomes to be in fluid communication with the hollow interior of the vial 404 in response to the piston 402 puncturing or piercing the seal member of the vial 404.

The body 412 includes a handle portion 420 configured to be handheld during coupling of the vial 404 and the drug delivery device 400 and during drug delivery. Flattened sides of the handle portion 420 include two facing finger openings 422 configured to receive a thumb of a user's hand with the index and middle finger of this hand on either side of a discharge connection 424 of the handle portion 420 on an outside of the handle portion's end wall 426 or elsewhere on the handle portion 420. By moving together the thumb and the two other fingers the vial 404 can be displaced with respect to the piston 402 or the body 412 and the drug consequently sprayed out of the vial 404 through the discharge channel 418 and through the opening 414. The handle portion 420, as well as various other aspects of the drug delivery device 400, are further described in U.S. Pat. No. 4,946,069 entitled "Dispenser For Manually Discharging Flowable Media" issued Aug. 7, 1990.

The discharge connection 424 includes a piston sleeve 428 constructed in one piece therewith and radially spaced within the discharge connection 424. The piston sleeve 428 projects beyond the end wall 426 both outwards and inwards into the handle portion 420. The piston sleeve 428 carries the piston 402 inserted therein. For this purpose the rear end of the piston 402 is extended to a piston shaft 430 constructed in one piece therewith and having substantially the same external cross-sections and which is set back with respect to the end face of the discharge connection 424 and with its associated end face engaging on an inner shoulder of the discharge connection 424 in such a way that a twisting device for the drug is formed between the end face and the discharge connection 424. In the vicinity of the piston 402, which only projects slightly beyond the piston sleeve 428, the outlet channel 418 is formed by a central longitudinal bore which, immediately behind the end of the piston sleeve 428 located at the piston 402, passes via a transverse bore into a main portion of the outlet channel 418, which is defined between an outer circumference of the piston shaft 430 and an inner circumference of the piston sleeve 428 and which can, e.g., be formed by at least one longitudinal groove on the outer circumference of the piston shaft 430.

A base plate at a distal end 404d of the vial 404 is curved inwards in such a way that it forms on its outside a thumb depression for the engagement of the thumb cap of the user's hand and on the inside has a correspondingly curved protuberance. A front end face of the piston 402 includes a depression that is shaped substantially identically to the protuberance and whose base face strikes against the cylinder base plate at the end of the complete pump stroke, so that virtually no residual quantity of the drug to be discharged remains in the vial 404 after actuation. The base plate is internal to the vial 404 and is thus obscured in FIGS. 6 and 6A. In other embodiments, the base plate is linear instead of curved.

One actuation of the vial 404 causes the drug to be delivered into one nostril of the patient into which the tip 416 has been inserted. Proper delivery of the drug in one dosing session can, however, require the drug to be delivered into each of the patient's nostrils. As mentioned above, it is desired that an equal dose of the drug be delivered to each nostril to inhibit under-dosing of the drug.

In order to allow for two drug sprays from the drug delivery device 400, the drug delivery device 400 includes a limiting device 432. Omitting the limiting device 432 allows the drug delivery device 400 to be actuated only one time to deliver one drug spray.

The limiting device 432 includes a plurality of successive stops in the proximal or stroke direction and which can be one stop larger or smaller than the number of partial strokes, so that at least there is such a stop 434 for all the partial strokes before the last partial stroke. A counter-stop defined by a proximal surface 404p of the vial 404 is associated with the stop 434 and successively located stops. The stop 434 includes two facing stop faces 436 in the form of inner shoulders that are curved and with which is associated as the counter-stop. The stop faces 436 and/or the counter-stop has a ramp 438 extending over at least part of its width and the stop faces 436 can be constructed as a ramp.

The stop 434 is formed by a circumferentially divided, radially inwardly projecting resilient collar 440, whose ring portions are in each case provided on a spring arm 442 freely projecting proximally in the direction of the pump stroke of the piston 402 or constructed in one piece therewith. In the illustrated embodiment there are two facing portions of the resilient collar 440 or spring arms 442 in the direction of the longer extension of the end wall 426 and are ring-segmental and whose ends are slightly set back with respect to the shallow curved longitudinal sides of the handle portion 420 and are consequently contact-free with respect to the body 412. The spring arms 442 are also curved by an arc angle of substantially 90° so that they form shell-shaped resilient, stable spring elements, which are relatively hard even in the case of relatively small wall thicknesses. The stop 434 or spring arms 442 form a component of a one-piece, muff-like stop body 444, which at its end remote from the free ends of the spring arms 442 has a ring disk-like end wall 446 spaced from the stop 434 and which in the vicinity of the connection of discharge connection 424 to its outside engages on a ring shoulder of the body 412 formed by the inside of the end wall 426. The stop body 444 is fixed by springing into a retaining clip 448, which is constructed in one piece with the body 412 and is formed by two ring segmental clip jaws, which project from the inside of the end wall 426. The clip jaws, which only engage around the stop body 444 in the vicinity of the spring arms 442 and also only over a small part of its length on the outside, are provided on their insides with in each case one ring groove or the like for the engagement of a locking bead 450, which is provided roughly in the plane of the inside of the end wall 426 on the outer circumference of the stop body 444 and serves for the resilient, axially secured springing of the stop body 444 into the retaining clip 448.

The stop 434 is spaced from the free ends of the spring arms 443. Between the stop face 436 and the free ends directed against the vial 404 in an initial position before first actuation, the spring arms 442 form on their facing inner faces centering guides 452 for the vial 404. The centering guides 452 are formed by an inner cone tapering in acute angled manner towards the stop face 436 and with which is associated as a counter-face an outer circumferential proximal edge of the open end of the vial 404, e.g., at the head portion 408 thereof.

At the end of the first stage of operation, the counter-stop face strikes against the stop face 436, so that a significant or at least noticeable resistance is exerted against further operation of the device 400. If this resistance is overcome by a correspondingly more powerful operation, then as a result of the ramp the spring arms 442 are forced outwards until they slide on the outer circumference of the vial 404 with the inner circumferential surfaces of the vial 404 connected to the ramp 438. Thus, on overcoming the stop face 436 the second stage of operation begins. At the end of the second stage of operation, the counter-stop face strikes against a further stop face 454 of the stop body 444. The further stop face 454 is appropriately formed by the inside of the end wall 446 and arranged in such a way that simultaneously the front end face of the piston 402 strikes against the vial's bottom plate. The end wall 446 is traversed by the piston sleeve 428 or the piston rod, so that it can contribute to the positional stabilization thereof.

As mentioned above, the collar 440 is resilient. The collar's resiliency allows the collar 440 to flex distally (downwardly in the view of the device 400 illustrated in FIGS. 4-6) in response to the vial 404 being pulled distally to remove the vial 404 from the device 400 after doses of the drug have been delivered. The collar's resiliency also allows the collar 440 to flex proximally (upwardly in the view of the device 400 illustrated in FIGS. 4-6) to allow a second vial to be used with the device 400 similar to that discussed above regarding the vial 404, and so on with one or more additional vials.

Embodiments of the drug delivery device 400 in the illustrated embodiment of FIGS. 4-6 are not powered, e.g., does not include any electrical components such as a processor, a sensor, a memory, a communications interface, etc. The drug delivery device 400 thus need not include a power supply though a power supply could be included, e.g., for connectivity, for powering a light source of the drug delivery device, etc.

The drug delivery device 400, as with every drug delivery device described herein as being configured to releasably couple to a vial, can be provided as part of a kit including a plurality of vials, e.g., two, three, four, five, six, seven, eight, nine, ten, etc., each configured to be successively releasably coupled to the drug delivery device 400. Each of the plurality of vials can be provided without yet being coupled to the drug delivery device 400, or one of the vials can be provided releasably coupled to the drug delivery device 400 with a remainder of the vials being provided without yet being coupled to the drug delivery device 400. Each of the plurality of vials can include an order indicator thereon that indicates an order in which the vial should be releasably coupled to the drug delivery device 400. For example, the order indicator can include a number printed on the vial, etched into the vial, printed on a label adhered to the vial, printed on a box or other package containing the vial therein, or otherwise made visible to a user of the vial. For another example, the order indicator can include a color on the vial, on a label on the vial, on a box or other package containing the vial therein, or otherwise made visible to a user of the vial. For yet another example, the order indicator can include a number and a color.

FIGS. 7 and 8 illustrate another exemplary embodiment of the drug delivery device 300 of FIG. 3. FIGS. 7 and 8 illustrate a drug delivery device 500 that is configured and used similar to the drug delivery device 400 of FIGS. 4-6 except that the drug delivery device 500 includes a device indicator 502 configured to present information to a user about a status of the drug delivery device 500. The device indicator 502 in this illustrated embodiment is configured to indicate whether or not the vial 404 is releasably coupled to the drug delivery device 500, e.g., is releasably coupled to a coupling mechanism 504 thereof. The device indicator 502 is a visual indicator, but as mentioned above the device indicator 502 can additionally or alternatively be an audio indicator configured to provide sound. The device indicator 502 is configured to indicate by color whether or not the vial 404 is releasably coupled to the drug delivery device 500. FIG. 7 shows the device indicator 502 as having a first color that is indicative of no vial being releasably coupled to the drug delivery device 500. FIG. 8 shows the device indicator 502 as having a second color that is different than the first color and that is indicative of the vial 404 being releasably coupled to the drug delivery device 500.

The drug delivery device 500 includes a single device indicator 502 in this illustrated embodiment but can include a plural number of device indicators 502. For example, a second device indicator 502 can be located on an opposite side of the drug delivery device 500 than the illustrated device indicator 502.

The location of the device indicator 502 in FIGS. 7 and 8 is shown as one example, as the device indicator 502 can be located in any of a variety of locations on the drug delivery device 500.

The drug delivery device 500 includes a movable mechanism operatively coupled to the device indicator 500 to cause the device indicator 500 to change between its two color states. For example, the movable mechanism can include an elongate rod that extends longitudinally along the coupling mechanism 502 and the drug delivery device's piston shaft that is configured to be pushed upward by the vial 404 as the vial 404 is advanced proximally along the coupling mechanism 502 to releasably couple to the drug delivery device 500. The elongate rod is spring biased or otherwise biased to a distal position such that when the vial 404 is removed from the drug delivery device 500, the elongate rod automatically moves distally to change the device indicator 502 from the second color back to the first color.

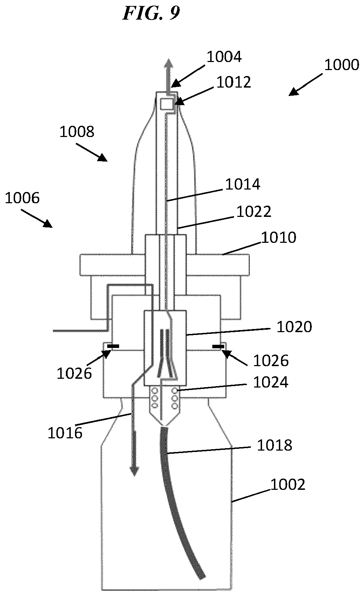

FIG. 9 illustrate another exemplary embodiment of the drug delivery device 300 of FIG. 3. FIG. 9 illustrates a drug delivery device 1000 that includes a coupling mechanism 1026 configured to releasably couple to a vial 1002. The coupling mechanism 1026 in this illustrated embodiment includes a groove formed in the drug delivery device 1000, and the vial 1002 includes a corresponding protrusion extending from the vial 1002 that is configured to be releasably seated in the groove. FIG. 9 illustrates the vial 1002 releasably coupled to the drug delivery device 1000. The vial 1002 is generally configured and used similar to the vial 100 of FIG. 1. The vial 1002 in this illustrated embodiment includes a base portion and a head portion that has a larger maximum outer diameter than then base portion, and the vial 1002 includes a tapering neck portion.

An opening 1004 of the nasal spray device 1000 through which the drug exits the nasal spray device 1000 is formed in in a dispensing head 1006 of the nasal spray device 1000 in a tip 1008 of the dispensing head 1006. The dispensing head 1006 includes a depth guide 1010. In an exemplary embodiment, the tip 1008 is configured to be inserted into a first nostril of the patient during a first stage of operation of the nasal spray device 1000 and into a second nostril of the patient during a second stage of operation of the nasal spray device 1000. The first and second stages of operation involve two separate actuations of the nasal spray device 1000, a first actuation corresponding to a first dose of the drug being delivered and a second actuation corresponding to a second dose of the drug being delivered. In some embodiments, the nasal spray device 1000 is configured to be actuated only once to deliver one nasal spray. In some embodiments, the nasal spray device 1000 is configured to be actuated three or more times to deliver three or more nasal sprays, e.g., four, five, six, seven, eight, nine, ten, etc.

In this illustrated embodiment, the dispensing head 1006 has a tapered shape in which the dispensing head 1006 has a smaller diameter at its distal end than at its proximal end where the opening 1004 is located. A spray chamber 1012 through which the drug is configured to pass before exiting the opening 1004 is located within a proximal portion of the tapered dispensing head 1006, distal to the opening 1004. When the drug passes through the spray chamber 1012 at speed, the spray chamber 1012 facilitates production of a fine mist that exits through the opening 10010 with a consistent spray pattern. Arrow 1014 illustrates a path of travel of the drug from the vial 1002 and out of the opening 1004.

In some embodiments, the dispensing head 1006 can include two tips 1008 each having an opening 1004 therein such that the nasal spray device 1000 is configured to simultaneously deliver doses of drug into two nostrils in response to a single actuation.

The dispensing head 1006 is configured to be pushed toward the vial 1002, e.g., depressed by a user pushing down on the depth guide 1010, to actuate the nasal spray device 1000. In other words, the dispensing head 1006 is configured as an actuator to be actuated to drive the drug from the vial 1002 and out of the nasal spray device 1000. In an exemplary embodiment, the nasal spray device 1000 is configured to be self-administered such that the user who actuates the nasal spray device 1000 is the patient receiving the drug from the nasal spray device 1000, although another person can actuate the nasal spray device 1000 for delivery into another person.

The actuation, e.g., depressing, of the dispensing head 1006 is configured to cause venting air to enter the vial 1002, as shown by a second arrow 1016. The air entering the vial 1002 displaces drug in the drug holder through a tube 1018 and then into a metering chamber 1020, which displaces drug proximally through a cannula 1022, through the spray chamber 1012, and then out of the opening 1004. In response to release of the dispensing head 1006, e.g., a user stops pushing downward on the dispensing head 1006, a bias spring 1024 causes the dispensing head 1006 to return to its default, resting position to position the dispensing head 1006 relative to the vial 1002 for a subsequent actuation and drug delivery.

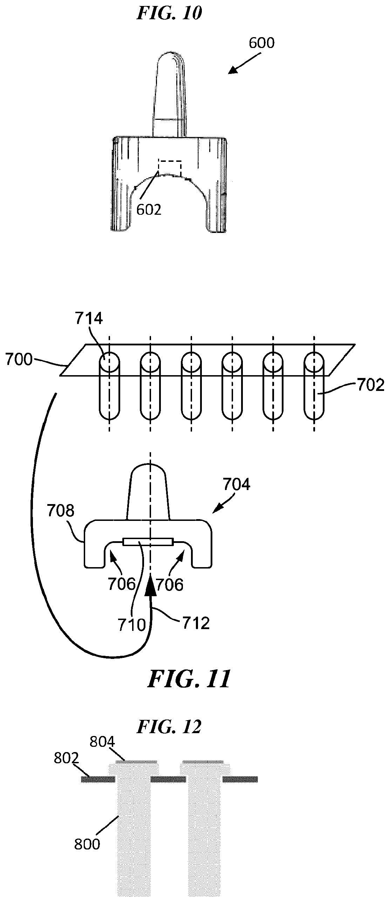

FIG. 10 illustrates another exemplary embodiment of the drug delivery device 300 of FIG. 3. FIG. 10 illustrates a drug delivery device 600 that is configured and used similar to the drug delivery device 400 of FIGS. 4-6 except that the drug delivery device 600 includes a coupling mechanism 602 that includes a cavity configured to receive the vial 404 therein. The coupling mechanism 602 also includes a piston located at least partially within the cavity that the vial 404 is configured to releasably engage within the cavity similar to that discussed above regarding the coupling mechanism 402. The coupling mechanism 602 being located within the drug delivery device 600 instead of extending therefrom, as in the embodiment of FIGS. 4-6, may help protect the coupling mechanism 602 from being damaged in transit and/or storage. For example, the coupling mechanism 602 can include threading on an internal surface thereof configured to releasably thread with threading on an external surface of the vial 404. For another example, the coupling mechanism 602 can include a clip configured to releasably clip to the head portion 408 of the vial 404. For yet another example, one of the coupling mechanism 602 and the vial 404 can include one or more protrusions extending therefrom configured to be releasably seated in one or more corresponding grooves or depressions formed in the other of the coupling mechanism 602 and the vial 404.

The drug delivery device 600 can include a device indicator similar to the device indicator 502 discussed above with respect to FIGS. 8 and 9.

In some embodiments, a plurality of vials each configured to be releasably coupled to a drug delivery device can be pre-loaded on a cartridge strip. Each of the vials is configured to be sequentially released from the cartridge strip for releasable coupling to the drug delivery device, as described herein. Pre-loading the vials on the cartridge strip may help a user know the correct order in which to couple to the vials to the drug delivery device because the vials can be lined up on the cartridge strip and simply be coupled to the drug delivery device in line order. In addition to or instead of the vials each including an order indicator, the cartridge strip can include an order indicator thereon that indicates an order in which the vials should be releasably coupled to a drug delivery device to provide further indication of vial order use. Pre-loading the vials on the cartridge strip may help a user comply with a prescribed dosing schedule and/or prescribed dosage amount, similar to a pill box with different compartments for each day's prescribed pills, because the cartridge strip of vials can provide a simple visible verification to a user of how many vials have already been coupled to the drug delivery device for use and how many vials have not yet been coupled to the drug delivery device for use. Pre-loading the vials on the cartridge strip may help prevent any of the vials from being lost before being coupled to the drug delivery device since the vials are all attached together via the cartridge strip.

FIG. 11 illustrates one embodiment of a cartridge strip 700 releasably coupled to a plurality of vials 702. Each of the vials 702 is generally configured and used similar to the vial 100 of FIG. 1. Six vials 702 are coupled to the cartridge strip 700 in this illustrated embodiment, but another number of vials 702 can be so coupled, e.g., two, three, four, five, seven, eight, nine, ten, etc.

FIG. 11 also illustrates another exemplary embodiment of the drug delivery device 300 of FIG. 3. FIG. 11 illustrates a drug delivery device 704 that is configured and used similar to the drug delivery device 400 of FIGS. 4-6. Opposed inner surfaces 706 of the drug delivery device's handle portion 708 serve as an alignment member configured to align the drug delivery device 704 relative to cartridge strip 700 by contacting the cartridge strip 700 so one of the vials 702 can be released from the cartridge strip 700 and releasably coupled to the drug delivery device 704 in proper alignment therewith, e.g., coupled to and in alignment with a coupling mechanism 710 of the drug delivery device 704. Arrow 712 indicates the proximal direction in which the vials 702 are sequentially moved into releasable coupling with the drug delivery device 704 by manual pushing of the one of the vials 702 that is aligned with the coupling mechanism 710. The coupling mechanism 710 can include, for example, a clip configured to releasably clip to the vial 702 when the vial 702 is pushed into engagement with the clip. For another example, one of the coupling mechanism 710 and the vial 702 can include one or more protrusions extending therefrom configured to be releasably seated in one or more corresponding grooves or depressions formed in the other of the coupling mechanism 710 and the vial 702 when the vial 702 has been pushed enough to cause engagement of the one or more protrusions and one or more corresponding grooves or depressions.

The drug delivery device 704 can be released from contact with the cartridge strip 700 before actuating the drug delivery device 704, e.g., before the vial 702 that is releasably coupled to the drug delivery device 704 is pushed proximally. For at least some users, the drug delivery device 704 can be released from the cartridge strip 700 before actuating the drug delivery device 704 may make the drug delivery device 704 easier to hold during drug delivery. Alternatively, the drug delivery device 704 can be coupled to the cartridge strip 700 during the actuation of the drug delivery device 704, e.g., when the vial 702 that is releasably coupled to the drug delivery device 704 is pushed proximally. The drug delivery device 704 remaining coupled to the cartridge strip 700 during drug delivery may make it easier and/or faster for a user to deliver drug from multiple ones of the vials, such as if a first vial is used to spray drug into one nostril of a patient and a second vial is used to spray drug into the patient's other nostril. The drug delivery device 704 remaining coupled to the cartridge strip 700 during drug delivery may help prevent inadvertent loss of one or the other of the drug delivery device 704 and the cartridge strip 700 if one or more of the vials 702 are used at different dosing times from one another, e.g., at a prescribed time separation between doses.

The drug delivery device 704 can include a device indicator similar to the device indicator 502 discussed above with respect to FIGS. 8 and 9.

In some embodiments, the drug delivery device 704 can be non-releasably coupled to the cartridge strip 700, which may make it easier and/or faster for a user to deliver drug from multiple ones of the vials and/or may help prevent inadvertent loss of one or the other of the drug delivery device 704 and the cartridge strip 700 if one or more of the vials 702 are used at different dosing times from one another. After all of the vials 702 have been used, e.g., after drug has been delivered therefrom using the drug delivery device 704, the used vials 702 and the drug delivery device 704 can be disposed of as medical waste in whole or in part or by recycling in whole or in part. Non-releasable coupling of the cartridge strip 700 and the drug delivery device 704 can be achieved in any of a variety of ways. For example, the cartridge strip 700 can have an elongate longitudinal channel formed in a side thereof, and the drug delivery device 704 can include at least one protrusion extending radially outward from the handle portion 708 that is configured to slide within the channel as the drug delivery device 704 is moved along the cartridge strip 700 to align with the different vials 702. Alternatively, the cartridge strip 700 can include the at least one protrusion, and the drug delivery device can include the elongate longitudinal channel. For another example, the handle portion 708 of the drug delivery device 704 can define an enclosed shape, with the opposed inner surfaces 706 defining at least a portion of the shape's perimeter, within which the cartridge strip 700 is located.

Each of the vials 702 in the embodiment of FIG. 11 includes a removable protective member or stopper 714 that is configured to be removed just prior to the vial 702 being releasably coupled to the drug delivery device 704, although as discussed above the vial 702 may omit the protective member or stopper 714.

FIG. 12 illustrates another embodiment in which each of a plurality of vials 800 releasably coupled to a cartridge strip 802 do not include a removable protective member or stopper but do include a seal member 804 at a proximal end thereof. Each of the vials 800 is generally configured and used similar to the vial 100 of FIG. 1. The cartridge strip 802 is generally configured and used similar to the cartridge strip 700 of FIG. 11. Two vials 800 are coupled to the cartridge strip 802 in this illustrated embodiment, but another number of vials 800 can be so coupled, e.g., three, four, five, six, seven, eight, nine, ten, etc.

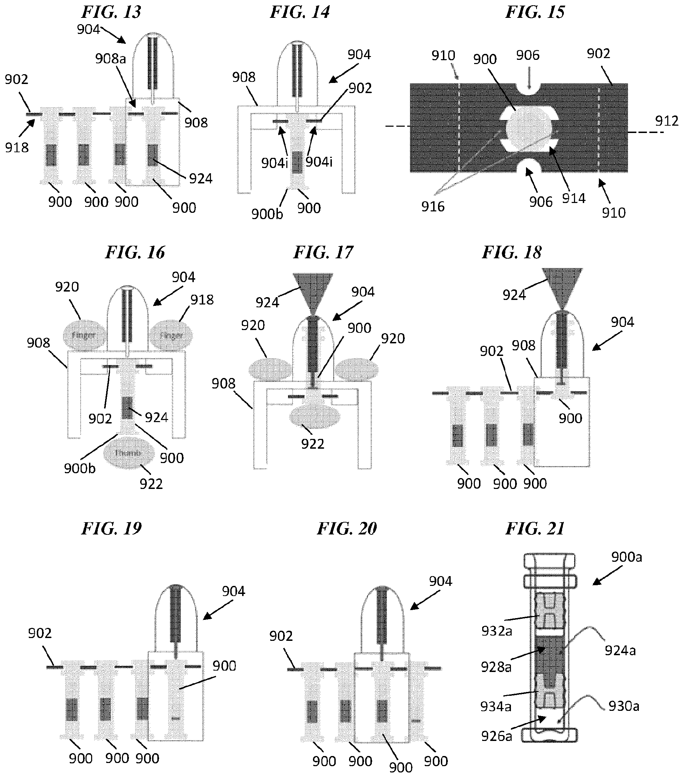

FIGS. 13 and 14 illustrate another embodiment in which each of a plurality of vials 900 are releasably coupled to a cartridge strip 902. Each of the vials 900 is generally configured and used similar to the vial 100 of FIG. 1. The cartridge strip 902 is generally configured and used similar to the cartridge strip 700 of FIG. 11. Four vials 900 are coupled to the cartridge strip 902 in this illustrated embodiment, but another number of vials 900 can be so coupled, e.g., two, three, five, six, seven, eight, nine, ten, etc.

FIGS. 13 and 14 also illustrate a drug delivery device 904 that is configured and used similar to the drug delivery device 400 of FIGS. 4-6. Opposed inner surfaces 904i of the drug delivery device's handle portion 908 serve as an alignment member configured to align the drug delivery device 904 relative to cartridge strip 902 so one of the vials 900 can be released from the cartridge strip 902 and releasably coupled to the drug delivery device 904 in proper alignment therewith, e.g., coupled to and in alignment with a coupling mechanism of the drug delivery device 904.

As shown in FIG. 15, the cartridge strip 902 includes a pair of detents 906 on opposed longitudinal sides of the cartridge strip 902 that is associated with each of the vials 900. In other words, the cartridge strip 902 in this illustrated embodiment includes four pairs of detents 906, one pair for each of the four vials 900. Each one of the vials 900, when coupled to the cartridge strip 902, is positioned between its associated pair of detents 906. Each pair of detents 906 serves as an alignment mechanism configured to cooperate with the alignment member of the drug delivery device 904 to align the drug delivery device 904 relative to the cartridge strip 902 so one of the vials 900 can be released from the cartridge strip 902 and releasably coupled to the drug delivery device 904 in proper alignment therewith. Each pair of detents 906 is configured to engage the opposed inner surfaces 904i of the drug delivery device 904, as shown in FIGS. 13 and 14. The engagement of the two detents 906 with the drug delivery device 904 allows the drug delivery device 904 to be properly aligned relative to cartridge strip 902 for vial 900 coupling. The detents 906 and the opposed inner surfaces 904i have complementary sizes and shapes to facilitate engagement therebetween.

The cartridge strip 902 includes a plurality of perforations 910 that each extend substantially perpendicular to a longitudinal axis 912 of the cartridge strip 902. A person skilled in the art will appreciate that the perforations 910 may not extend precisely perpendicular to the longitudinal axis 912 but nevertheless be considered to be substantially perpendicular thereto due to any number of factors, such as manufacturing tolerances and sensitivity of measurement equipment. A number of the perforations 910 is one less than a number of vials 900 that can be coupled to the cartridge strip 902. The cartridge strip 902 in this illustrated embodiment thus includes three perforations 910, which is one less than the four vials 900. The perforations 910 are configured to allow for tearing of the cartridge strip 902 therealong. After one of the vials 900 has been coupled to the drug delivery device 904 and removed from the cartridge strip 902, the cartridge strip 902 can be torn along the one of the perforations 910 adjacent to the vial's original coupling location with the cartridge strip 902 and nearest a free end of the cartridge strip 902. For example, perforation 910a labeled in FIG. 13 indicates the perforation 910 that can be torn after the right-most vial 900 has been coupled to the drug delivery device 904 and removed from the cartridge strip 902. The cartridge strip 902 can be torn at a perforation 910 without the vial 900 coupled to the cartridge strip 902, or the perforation 910 can be torn with the vial 900 re-coupled to the cartridge strip 902 after having been coupled to and used with the drug delivery device 904.

The cartridge strip 902 includes an opening 914 therein for each of the vials 900 and includes a pair of tabs 916 that extends radially inward into each of the openings 914. In other words, the cartridge strip 902 in this illustrated embodiment includes four openings 914 and four pairs of tabs 916, one opening and one pair for each of the four vials 900. Each one of the vials 900, when coupled to the cartridge strip 902, is engaged with one of the pairs of tabs 916. Each pair of tabs 916 serves as a coupling feature configured to cooperate with a coupling mechanism of its associated vial 900 to releasably couple the vial 900 to the cartridge strip 902. The coupling mechanism of each vial 900 includes a rib 918 in a head portion of the vial 900. The rib 918 is configured to releasably seat the tabs 916 therein, as shown in FIGS. 13-15. The rib 918 extends circumferentially around the vial's head portion, which may facilitate coupling of the vial 900 at any rotational orientation relative to the cartridge strip 902. Each of the vials 900 also includes a coupling mechanism configured to couple the vial 900 to the drug delivery device 904, as discussed herein.

In this illustrated embodiment, the cartridge strip 902 is resilient. The cartridge strip's resiliency allows the cartridge strip 902 to flex to facilitate coupling each of the vials 900 with a drug delivery device 904. The cartridge strip 902 is made of one or more materials that facilitate the flexing of the cartridge strip 902, such as the cartridge strip 902 being made of at least one plastic. In this illustrated embodiment, the entire cartridge strip 902 is resilient. In other embodiments, only a portion of the cartridge strip 902 is resilient. Only a portion of the cartridge strip 902 being resilient with a remainder of the cartridge strip 902 being rigid and not resilient may facilitate user handling of the cartridge strip 902 at least during coupling of the vials 900 with the drug delivery device 904. For example, the tabs 916 can be resilient with a remainder of the cartridge strip 902 not being resilient. In such embodiments the cartridge strip 902 lacks the perforations 910. For another example, the tabs 916 and areas adjacent each of the perforations 910 can be resilient with a remainder of the cartridge strip 902 not being resilient.

In an exemplary embodiment of using the cartridge strip 902, the drug delivery device 904 is positioned relative to the cartridge strip 902 with the drug delivery device's opposed inner surface 904i engaging one of the cartridge strip's pair of detents 906, as shown in FIGS. 13 and 14. The drug delivery device 904 is thereby properly aligned with one of the vials 900 for coupling. The one of the vials 900 is then releasably coupled to the drug delivery device 904. As shown in FIG. 16, a user can position two fingers 920 on a proximal surface of the drug delivery device's handle portion 908 and a thumb 922 on a distal surface 900d of the one of the vials 900. Then, as shown in FIGS. 17 and 18, the user can push the vial 900 proximally with their thumb 920 while holding the drug delivery device 904 steady with their fingers 920 to couple the vial 900 to the drug delivery device 904 and cause delivery of a drug 924 from the vial 900 and out of the drug delivery device 904. The drug delivery device 904 remains engaged with the detents 906 during delivery of the drug 924. The vial 900 includes a distal flange 900b configured to facilitate a user's proximal pushing of the vial 900 by providing an enlarged surface area for the user's thumb 922 to push against.

After the drug 924 has been delivered from the drug delivery device 904, the vial 900 can be re-coupled to the cartridge strip 902 with the rib 918 again engaging the tabs 916. As shown in FIG. 19, the vial 900 can be pulled distally relative to the drug delivery device 904 and the cartridge strip 902 to re-couple the ribs 918 and the tabs 916. The vial's distal flange 900b is also configured to facilitate a user's grip of the vial's distal end to facilitate the user pulling the vial 900 distally. The drug delivery device 904 can then be repositioned relative to the cartridge strip 902 in engagement with another pair of detents 906 to prepare the drug delivery device 904 to be coupled to and used with another one of the vials 900, as shown in FIG. 20. Each of the remaining vials 900 can be similarly coupled to and used with the drug delivery device 904.

The vial 900 can include a hollow interior within a base portion of the vial that includes a single chamber containing the drug 924 therein similar to the vial 100 of FIG. 1, as in the illustrated embodiment of FIGS. 13-20. Alternatively, a hollow interior in a base portion of the vial 900 can include two chambers. FIG. 21 illustrates an exemplary embodiment of the vial 900 as a vial 900a including two chambers.

As shown in FIG. 21, a base portion of the vial 900a defines a distal cavity 926a and a proximal cavity 928a. Air 930a is contained in the distal cavity 926a, and a drug 924a is contained in the proximal cavity 928a. In this way, the drug 924a can exit the vial 900a for delivery to a user followed by exit of the air 930a from the vial 900a for delivery to the user. The air 903a exiting the vial 900a after the drug 924a may help ensure that substantially all of the drug 924a has exited the drug holder 900a such that substantially no residual drug remains in the drug holder 900a. A person skilled in the art will appreciate that the amount of residual drug may not be precisely zero but nevertheless be considered to be substantially none due to any number of factors, such as sensitivity of measurement equipment. Having substantially no residual drug left in a vial after use thereof may prevent access to the drug after use of the vial, which may be particularly important for esketamine, ketamine, and other controlled substances that could be abused and/or be more likely than other drugs to lead to an addiction. Having substantially no residual drug left in a vial after use thereof may facilitate recycling of the used vial (alone or in combination with all of part of a drug delivery device that delivered drug from the vial) since substantially no drug will be present when the vial is recycled. Some drugs, such as esketamine, ketamine, and other controlled substances, may be required to be disposed of per the drug's Risk Evaluation and Mitigation Strategies (REMS) to help, e.g., ensure that the drug is not accessed by an unauthorized person. Disposal of the used vial can include recycling or throwing out the used vial (alone or in combination with all of part of a drug delivery device that delivered drug from the vial) as medical waste. Having substantially no residual drug left in a vial after use thereof may help satisfy the REMS since the vial will be substantially free of the drug when the vial is disposed.

The vial 900a includes a first seal member 932a configured to provide a fluid tight seal at a proximal end of the proximal cavity 928a such that the drug 924a is contained in the proximal cavity 928a in the vial 900a until the seal provided by the first seal member 932a is broken. The first seal member 932a is located in the base portion of the vial 900a in this illustrated embodiment but can instead be located in a head portion of the vial 900a. The seal provided by the first seal member 932a can be broken in a variety of ways, such as by being pierced by a needle, pin, piston, etc. of the drug delivery device to which the vial 900a is coupled. The first seal member 932a can have a variety of configurations, as will be appreciated by a person skilled in the art, such as by being a pierceable polymer septum or a foil layer. The first seal member 932a can be protected from accidental puncturing or piercing before intended use with a removable protective member or stopper, such as a TE seal, etc. located at the proximal end of the vial 200.

The distal and proximal cavities 926a, 928a are isolated from one another in an initial state of the vial 900a. The vial 900a includes a second seal member 934a that is located in the base portion. The second seal member 934a is configured to provide a fluid tight seal such that the air 930a in the distal cavity 926a and the drug 924a in the proximal cavity 928a are separated from each other until the seal provided by the second seal member 934a is broken. The seal provided by the second seal member 934a can be broken in a variety of ways, such as by being pierced by a needle, pin, piston, etc. of the drug delivery device to which the vial 900a is coupled, such as the same needle, pin, piston, etc. that breaks the first seal member 932a. The second seal member 934a can have a variety of configurations, as will be appreciated by a person skilled in the art, such as by being a pierceable polymer septum or a foil layer.

The first seal member 932a is proximal to both of the distal and proximal cavities 926a, 928a. The second seal member 934a is located between the distal and proximal cavities 926a, 928a, e.g., is distal to the proximal cavity 928a and is proximal to the distal cavity 926a, such that the distal and proximal cavities 926a, 928a are isolated from each other prior to piercing or puncturing of the second seal member 934a.

Embodiments of nasal drug delivery devices disclosed herein can be designed to be disposed of after a single use, or they can be designed to be used multiple times. In either case, in at least some embodiments, the drug delivery device can be reconditioned for reuse after at least one use. Reconditioning can include any combination of the steps of disassembly of the drug delivery device, followed by cleaning or replacement of particular pieces and subsequent reassembly. In particular, the drug delivery device can be disassembled, and any number of the particular pieces or parts of the drug delivery device can be selectively replaced or removed in any combination. Upon cleaning and/or replacement of particular parts, the drug delivery device can be reassembled for subsequent use either at a reconditioning facility, or by a health care provider immediately prior to use. A person skilled in the art will appreciate that reconditioning of a drug delivery device can utilize a variety of techniques for disassembly, cleaning/replacement, and reassembly. Use of such techniques, and the resulting reconditioned drug delivery device, are all within the scope of the present application.

The present disclosure has been described above by way of example only within the context of the overall disclosure provided herein. It will be appreciated that modifications within the scope of the claims may be made without departing from the overall scope of the present disclosure. The invention is defined in the following claims.

## Claims

1. A drug delivery device (400, 600, 704, 904) comprising:
a tip (416) configured to be positioned in a nose of a patient, the tip having an opening (414) therein;
a coupling mechanism (410,602,710) configured to releasably couple to a first vial (100,702, 800,900) containing a first drug therein, to release the first vial therefrom, and to subsequently be releasably coupled to a second vial containing a second drug therein;
a plunger (402) configured to be actuated in a stroke direction through a first partial stroke and a second partial stroke to cause the first drug to exit through the opening, and to be subsequently actuated in the stroke direction through the first partial stroke and the second partial stroke to cause the second drug to exit through the opening;
**characterized in that** the drug delivery device further comprises:
a limiting device (432) comprising:
a one-piece stop body (444);
a plurality of spring arms (442) freely projecting in the stroke direction from the stop body and forming a component of the stop body;
a stop (434) formed by a circumferentially divided, radially inwardly projecting resilient collar (440), portions of which are in each case provided on each of the plurality of spring arms (442), the stop including a plurality of facing stop faces (436,454) in the form of an inner shoulder of the resilient collar (440); and
wherein the stop face is configured such that in use a counter-stop defined by a proximal surface (404p) of the first or second vial at the end of the first partial stroke strikes against the stop face (436), so that a noticeable resistance is exerted against further operation of the device, and
wherein the spring arms (442) are configured to be forced outwards until they slide on the outer circumference of the first or second vial, thereby allowing the resistance to be overcome by a correspondingly more powerful actuation.

2. The device of claim 1, wherein the coupling mechanism (410) includes any one of: threading on the plunger; a clip; one or more protrusions extending radially outward from the plunger; and one or more grooves or depressions formed in the plunger.

3. The device of claim 1, further comprising a handle portion (420); wherein the coupling mechanism (410) 2. includes threading on an inner surface of a cavity formed in the handle portion.

4. A drug delivery system, comprising:
a first vial (100) containing a first drug therein;
a second vial (100) containing a second drug therein; and
the nasal drug delivery device (400) of any of claims 1 to 3

5. The system of claim 4, further comprising one or more additional vials each containing a drug therein and each being configured to sequentially releasably couple to the coupling mechanism (410) after the second vial has been released from the coupling mechanism (410);
wherein, for each of the one or more additional vials, the plunger (402) is configured to be actuated to cause the drug contained in the additional via to exit through the opening.

6. The device or system of any of any preceding claim, wherein the plunger (402) is configured to be actuated again after the first drug exits through the opening and before the first vial is released from the coupling mechanism (410) to again cause the first drug to exit through the opening

7. The device or system of any preceding claim, wherein the plunger (402) is configured to be actuated again after the second drug exits through the opening and before the second vial is released from the coupling mechanism (410) to again cause the second drug to exit through the opening.

8. The system according to any of claims 4 to 7, wherein the coupling mechanism (410) includes threading on the plunger (402); and each of the first and second vials includes threading configured to thread with the threading on the plunger.

9. The system according to any of claims 4 to 7, wherein the coupling mechanism (410) includes a clip configured to clip to each of the first and second vials.

10. The system according to any of claims 4 to 7, wherein the coupling mechanism (410) includes one or more protrusions extending radially outward from the plunger (402); and
each of the first and second vials includes one or more grooves or depressions configured to be releasably seated in the one or more protrusions.

11. The system according to any of claims 4 to 7, wherein the coupling mechanism (410) includes one or more grooves or depressions formed in the plunger (402); and
each of the first and second vials includes one or more grooves or depressions in which the one or more protrusions are configured to be releasably seated.

12. The system according to any of claims 4 to 7 when depending on claim 3, wherein each of the first and second vials includes threading on an external surface thereof that is configured to thread with the threading on the inner surface of the cavity.

13. The system according to any of claims 4 to 12, further comprising a cartridge strip (700) releasably coupled to each of the first and second vials prior to the coupling mechanism (410,710) being releasably coupled to the first vial, or wherein each of the first and second vials are loose from one another prior to the coupling mechanism being releasably coupled to the first vial.

14. The system according to any of claims 4 to 13, wherein the first drug is one of ketamine, esketamine, naloxone, and sumatriptan; and
the second drug is one of ketamine, esketamine, naloxone, and sumatriptan.

15. The system according to any of claims 4 to 14, wherein the first drug and the second drug are the same, or wherein the first drug and the second drug are different.

## Patentansprüche

1. Arzneimittelabgabevorrichtung (400, 600, 704, 904), umfassend:
eine Spitze (416), die konfiguriert ist, um in einer Nase eines Patienten positioniert zu werden, wobei die Spitze (414) eine Öffnung darin aufweist;
einen Kopplungsmechanismus (410, 602, 710), der so konfiguriert ist, dass er sich lösbar mit einem ersten Fläschchen (100, 702, 800, 900), das ein erstes Arzneimittel darin enthält, koppelt, um das erste Fläschchen davon freizugeben, und um anschließend lösbar mit einem zweiten Fläschchen, das ein zweites Arzneimittel darin enthält, gekoppelt zu werden;
einen Kolben (402), der so konfiguriert ist, dass er in einer Hubrichtung durch einen ersten Teilhub und einen zweiten Teilhub betätigt wird, um zu bewirken, dass das erste Arzneimittel durch die Öffnung austritt, und dass er anschließend in der Hubrichtung durch den ersten Teilhub und den zweiten Teilhub betätigt wird, um zu bewirken, dass das zweite Arzneimittel durch die Öffnung austritt;
**dadurch gekennzeichnet, dass** die Arzneimittelverabreichungsvorrichtung ferner umfasst:
eine Begrenzungsvorrichtung (432), umfassend:
einen einteiligen Anschlagkörper (444);
eine Vielzahl von Federarmen (442), die frei in der Hubrichtung von dem Anschlagkörper vorstehen und eine Komponente des Anschlagkörpers bilden;
einen Anschlag (434), der durch einen umfangsmäßig geteilten, radial nach innen vorstehenden elastischen Kragen (440) gebildet ist, von dem jeweils Abschnitte an jedem der Vielzahl von Federarmen (442) vorgesehen sind, wobei der Anschlag eine Vielzahl von einander zugewandten Anschlagflächen (436, 454) in Gestalt einer inneren Schulter des elastischen Kragens (440) einschließt;
und
wobei die Anschlagfläche so konfiguriert ist, dass im Gebrauch ein Gegenanschlag, der durch eine proximale Oberfläche (404p) des ersten oder zweiten Fläschchens am Ende des ersten Teilhubs definiert ist, gegen die Anschlagfläche (436) schlägt, so dass ein spürbarer Widerstand gegen einen weiteren Betrieb der Vorrichtung ausgeübt wird, und
wobei die Federarme (442) so konfiguriert sind, dass sie nach außen gedrückt werden, bis sie auf dem Außenumfang des ersten oder zweiten Fläschchens gleiten, wodurch der Widerstand durch eine entsprechend stärkere Betätigung überwunden werden kann.

2. Vorrichtung nach Anspruch 1, wobei der Kopplungsmechanismus (410) eines der Folgenden einschließt: ein Gewinde auf dem Kolben; eine Klammer; einen oder mehrere Vorsprünge, die sich von dem Kolben radial nach außen erstrecken; und eine oder mehrere Rillen oder Vertiefungen, die in dem Kolben ausgebildet sind.

3. Vorrichtung nach Anspruch 1, ferner umfassend einen Griffabschnitt (420);
wobei der Kopplungsmechanismus (410) ein Gewinde auf einer Innenfläche eines in dem Griffabschnitt ausgebildeten Hohlraums einschließt.

4. Arzneimittelabgabevorrichtung, umfassend:
ein erstes Fläschchen (100), das ein erstes Arzneimittel enthält;
ein zweites Fläschchen (100), das ein zweites Arzneimittel enthält; und
die nasale Arzneimittelabgabevorrichtung (400) nach einem der Ansprüche 1 bis 3.

5. System nach Anspruch 4, das ferner ein oder mehrere zusätzliche Fläschchen umfasst, die jeweils ein Arzneimittel enthalten und jeweils so konfiguriert sind, dass sie sich nacheinander lösbar mit dem Kopplungsmechanismus (410) verbinden, nachdem das zweite Fläschchen aus dem Kopplungsmechanismus (410) gelöst worden ist;
wobei für jedes des ein oder mehreren zusätzlichen Fläschchen der Kolben (402) so konfiguriert ist, dass er betätigt wird, um zu bewirken, dass das in dem zusätzlichen Fläschchen enthaltene Arzneimittel durch die Öffnung austritt.

6. Vorrichtung oder System nach einem der vorhergehenden Ansprüche, wobei der Kolben (402) so konfiguriert ist, dass er erneut betätigt wird, nachdem das erste Arzneimittel durch die Öffnung ausgetreten ist und bevor das erste Fläschchen von dem Kupplungsmechanismus (410) gelöst wird, um erneut zu bewirken, dass das erste Arzneimittel durch die Öffnung austritt.

7. Vorrichtung oder System nach einem der vorhergehenden Ansprüche, wobei der Kolben (402) so konfiguriert ist, dass er erneut betätigt wird, nachdem das zweite Arzneimittel durch die Öffnung ausgetreten ist und bevor das zweite Fläschchen von dem Kupplungsmechanismus (410) gelöst wird, um erneut zu bewirken, dass das zweite Arzneimittel durch die Öffnung austritt.

8. System nach einem der Ansprüche 4 bis 7, wobei der Kopplungsmechanismus (410) ein Gewinde auf dem Kolben (402) aufweist; und
jedes der ersten und zweiten Fläschchen ein Gewinde einschließt, das so konfiguriert ist, dass es mit dem Gewinde auf dem Kolben verschraubt wird.

9. System nach einem der Ansprüche 4 bis 7, wobei der Kopplungsmechanismus (410) eine Klammer einschließt, die so konfiguriert ist, dass sie an jedem der ersten und zweiten Fläschchen befestigt angeklammert wird.

10. System nach einem der Ansprüche 4 bis 7, wobei der Kopplungsmechanismus (410) ein oder mehrere Vorsprünge einschließt, die sich von dem Kolben (402) radial nach außen erstrecken; und
jedes der ersten und zweiten Fläschchen eine oder mehrere Rillen oder Vertiefungen einschließt, die so konfiguriert sind, dass sie lösbar in den ein oder mehreren Vorsprüngen sitzen.

11. System nach einem der Ansprüche 4 bis 7, wobei der Kopplungsmechanismus (410) eine oder mehrere Rillen oder Vertiefungen einschließt, die in dem Kolben (402) ausgebildet sind; und
jedes der ersten und zweiten Fläschchen eine oder mehrere Rillen oder Vertiefungen einschließt, in denen die ein oder mehreren Vorsprünge so konfiguriert sind, dass sie lösbar eingesetzt werden.

12. System nach einem der Ansprüche 4 bis 7, in Abhängigkeit von Anspruch 3, wobei
jedes der ersten und zweiten Fläschchen ein Gewinde auf einer Außenfläche davon einschließt, das so konfiguriert ist, dass es mit dem Gewinde auf der Innenfläche des Hohlraums ineinander greift.

13. System nach einem der Ansprüche 4 bis 12, ferner umfassend einen Kartuschenstreifen (700), der lösbar mit jedem der ersten und zweiten Fläschchen gekoppelt ist, bevor der Kopplungsmechanismus (410, 710) lösbar mit dem ersten Fläschchen gekoppelt wird, oder wobei jedes der ersten und zweiten Fläschchen lose voneinander sind, bevor der Kopplungsmechanismus lösbar mit dem ersten Fläschchen gekoppelt wird.

14. System nach einem der Ansprüche 4 bis 13, wobei das erste Arzneimittel eines von Ketamin, Esketamin, Naloxon und Sumatriptan ist; und
das zweite Arzneimittel eines von Ketamin, Esketamin, Naloxon und Sumatriptan ist.

15. System nach einem der Ansprüche 4 bis 14, wobei das erste Arzneimittel und das zweite Arzneimittel das gleiche sind oder wobei das erste Arzneimittel und das zweite Arzneimittel unterschiedlich sind.

## Revendications

1. Dispositif d'administration de médicament (400, 600, 704, 904), comprenant :
un embout (416) conçu pour être positionné dans le nez d'un patient, l'embout ayant une ouverture (414) ;
un mécanisme d'accouplement (410, 602, 710) conçu pour s'accoupler de manière libérable à un premier flacon (100, 702, 800, 900) contenant un premier médicament, pour libérer le premier flacon de celui-ci et pour être ensuite accouplé de manière libérable à un second flacon contenant un second médicament ;
un piston (402) conçu pour être actionné dans un sens de course pendant une première course partielle et une seconde course partielle afin d'amener le premier médicament à sortir par l'ouverture, et pour être ensuite actionné dans le sens de course pendant la première course partielle et la seconde course partielle afin d'amener le second médicament à sortir par l'ouverture ;
**caractérisé en ce que** le système d'administration de médicament comprend en outre :
un dispositif de limitation (432), comprenant :
un corps de butée monobloc (444) ;
une pluralité de bras à ressort (442) faisant librement saillie dans le sens de course à partir du corps de butée et formant un composant du corps de butée ;
une butée (434) formée par une collerette élastique (440) divisée circonférentiellement et faisant saillie radialement vers l'intérieur, dont des parties sont dans chaque cas pourvues sur chacun de la pluralité de bras à ressort (442), la butée comportant une pluralité de faces de butée en regard (436, 454) sous la forme d'un
épaulement interne de la collerette élastique (440) ; et
dans lequel la face de butée est conçue de telle sorte qu'en cours d'utilisation, une contre-butée définie par une surface proximale (404p) du premier ou du second flacon à la fin de la première course partielle frappe contre la face de butée (436), de telle sorte qu'une résistance notable est exercée contre la poursuite du fonctionnement du dispositif, et
dans lequel les bras à ressort (442) sont conçus pour être forcés vers l'extérieur jusqu'à ce qu'ils glissent sur la circonférence externe du premier ou du second flacon, permettant ainsi à la résistance d'être surmontée par un actionnement correspondant plus puissant.

2. Dispositif selon la revendication 1, dans lequel le mécanisme d'accouplement (410) comporte l'un parmi : un filetage sur le piston ; un clip ; une ou plusieurs protubérances s'étendant radialement vers l'extérieur à partir du piston ; et une ou plusieurs rainures ou dépressions formées dans le piston.

3. Dispositif selon la revendication 1, comprenant en outre une partie poignée (420) ;
dans lequel le mécanisme d'accouplement (410) comporte un filetage sur une surface interne d'une cavité formée dans la partie poignée.

4. Système d'administration de médicament, comprenant :
un premier flacon (100) contenant un premier médicament ;
un second flacon (100) contenant un second médicament ; et
le dispositif d'administration de médicament (400) nasal selon l'une quelconque des revendications 1 à 3.

5. Système selon la revendication 4, comprenant en outre un ou plusieurs flacons supplémentaires contenant chacun un médicament et conçus pour s'accoupler séquentiellement de manière libérable au mécanisme d'accouplement (410) après que le second flacon a été libéré du mécanisme d'accouplement (410) ;
dans lequel, pour chacun du ou des flacons supplémentaires, le piston (402) est conçu pour être actionné pour amener le médicament contenu dans le flacon supplémentaire à sortir par l'ouverture.

6. Dispositif ou système selon l'une quelconque revendication précédente, dans lequel le piston (402) est conçu pour être actionné à nouveau après que le premier médicament est sorti par l'ouverture et avant que le premier flacon ne soit libéré du mécanisme d'accouplement (410) pour amener à nouveau le premier médicament à sortir par l'ouverture.

7. Dispositif ou système selon l'une quelconque revendication précédente, dans lequel le piston (402) est conçu pour être actionné à nouveau après que le second médicament est sorti par l'ouverture et avant que le second flacon ne soit libéré du mécanisme d'accouplement (410) pour amener à nouveau le second médicament à sortir par l'ouverture.

8. Système selon l'une quelconque des revendications 4 à 7, dans lequel le mécanisme d'accouplement (410) comporte un filetage sur le piston (402) ; et
chacun des premier et second flacons comporte un filetage conçu pour s'enfiler avec le filetage sur le piston.

9. Système selon l'une quelconque des revendications 4 à 7, dans lequel le mécanisme d'accouplement (410) comporte un clip conçu pour s'attacher à chacun des premier et second flacons.

10. Système selon l'une quelconque des revendications 4 à 7, dans lequel le mécanisme d'accouplement (410) comporte une ou plusieurs protubérances s'étendant radialement vers l'extérieur à partir du piston (402) ; et
chacun des premier et second flacons comporte une ou plusieurs rainures ou dépressions conçues pour être placées de manière libérable dans la ou les protubérances.

11. Système selon l'une quelconque des revendications 4 à 7, dans lequel le mécanisme d'accouplement (410) comporte une ou plusieurs rainures ou dépressions formées dans le piston (402) ; et
chacun des premier et second flacons comporte une ou plusieurs rainures ou dépressions dans lesquelles la ou les protubérances sont conçues pour être placées de manière libérable.

12. Système selon l'une quelconque des revendications 4 à 7 lorsqu'elles dépendent de la revendication 3, dans lequel
chacun des premier et second flacons comporte un filetage sur une surface extérieure de celui-ci qui est conçu pour s'enfiler avec le filetage sur la surface interne de la cavité.

13. Système selon l'une quelconque des revendications 4 à 12, comprenant en outre une bande à cartouche (700) accouplée de manière libérable à chacun des premier et second flacons avant que le mécanisme d'accouplement (410, 710) ne soit accouplé de manière libérable au premier flacon, ou dans lequel chacun des premier et second flacons sont séparés l'un de l'autre avant que le mécanisme d'accouplement ne soit accouplé de manière libérable au premier flacon.

14. Système selon l'une quelconque des revendications 4 à 13, dans lequel le premier médicament est l'un parmi kétamine, eskétamine, naloxone et sumatriptan ; et
le second médicament est l'un parmi kétamine, eskétamine, naloxone et sumatriptan.

15. Système selon l'une quelconque des revendications 4 à 14, dans lequel le premier médicament et le second médicament sont identiques, ou dans lequel le premier médicament et le second médicament sont différents.
